(19) Europäisches Patentamt
European Patent Office
Office européen des brevets

(11) **EP 3 643 293 A1**

(12) **EUROPEAN PATENT APPLICATION**

(43) Date of publication:
**29.04.2020 Bulletin 2020/18**

(21) Application number: **19382848.0**

(22) Date of filing: **02.10.2019**

(51) Int Cl.:
*A61K 8/34* (2006.01)      *A61K 8/63* (2006.01)
*A61K 8/97* (2017.01)      *A61K 36/36* (2006.01)
*A61P 17/00* (2006.01)      *A61Q 19/00* (2006.01)
*A61K 8/92* (2006.01)      *A61K 8/9789* (2017.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.10.2018 ES 201831041**

(71) Applicant: **E Grau Active Cosmetics, S.L.U.**
**08029 Barcelona (ES)**

(72) Inventor: **GRAU MONJO, Elena**
**08029 BARCELONA (ES)**

(74) Representative: **Sugrañes Patentes y Marcas**
**Calle Provenza 304**
**08008 Barcelona (ES)**

(54) **AN OLIVE OIL-BASED CONCENTRATE AND A TOPICALLY APPLIED PREPARATION FOR SKIN CARE AND REPAIR COMPRISING SAID CONCENTRATE**

(57)      An olive oil-based concentrate to be used as an active ingredient in a topically applied preparation for skin care and repair which comprises olive oil, with an ORAC factor of at least 8 ($\mu$mol Trolox equivalents/g), enriched with at least one extract from the olive fruit rich in maslinic acid and with at least one extract also from the olive fruit or leaf rich in Hydroxytyrosol. The invention also relates to a topically applied preparation for skin care and repair comprising between 1% and 3% by weight of the concentrate.

**Fig. 1**

EP 3 643 293 A1

**Description**

Technical field of the invention

The invention relates to the field of concentrates which are used as active ingredients for the preparation of topically applied creams or the like for skin care or for preventing or treating skin or dermatological diseases.

Background of the invention

The properties of olive oil in maintaining skin integrity are well documented at present. The practice of including olive oil in the formulation of creams, lotions and body milk is not something new, for example, for moisturising skin and preventing the appearance of wrinkles; for adding shine to hair and even as a base for therapeutic massages.

On the one hand, it is known that olive oil is an antioxidant product itself, its role in reducing oxidative stress having been demonstrated, which makes it a product of interest in the field of cosmetics for strengthening the function of the skin as a barrier and for reducing transepidermal water loss, improving the hydrolipid layer and the hydration levels of the skin.

Antioxidant vitamins found in olive oil, mainly Vitamin E, play an important role in this reduction of oxidative stress because they help fix free radicals (unstable molecules which, for various reasons such as sun exposure or environmental pollution, lack an electron, destabilising and creating reactions that can damage the cell membrane) thus contributing to the delay of skin aging.

On the other hand, the antioxidant effect of 3,3-dihydroxyphenylethanol is also well documented, hereinafter referred to as Hydroxytyrosol. To take advantage of this antioxidant action, Hydroxytyrosol extracts are ingredients used in the field of cosmetics. For example, Hydroxytyrosol extract is a product of great use in the formulation of sunscreens given that, thanks to its exclusive properties, it is one of the ingredients with greater capacity to combat sun damage to the skin.

It is the case that Hydroxytyrosol is a component, although a minority one, of the aqueous fraction of the olive, which is present in extra virgin olive oil (EVOO) but not in the rest of the oils, since it is eliminated with refining. To obtain high purity Hydroxytyrosol extracts there are different documented methods which are more or less complex. Today, there are numerous suppliers of Hydroxytyrosol concentrates on the market.

2-alpha, 3-betadihydroxy-28-carboxyoleanan, hereinafter referred to as maslinic acid, is an acid that not very common in nature, having been detected in several plants. Its antihistamine and anti-inflammatory effect is well documented. It is possible to obtain extracts of maslinic acid from waste products of the milling of the olive, especially the waxes on the surface of the Olea europaea fruit, thus obtaining high yields and a high purity of the resulting concentrates.

To take advantage of the properties of maslinic acid, for example, the cosmetic use of maslinic acid, a salt thereof, a derivative thereof, or a mixture thereof is known for the redensification of the skin, as well as dermocosmetic compositions for the redensification of the skin comprising maslinic acid. Examples of cosmetic uses of maslinic acid are described in EP 1295587 and ES 2462565.

Transepidermal water loss, which is mentioned above and also called TEWL, is defined as the amount of water that passes from the inside of the body to the atmosphere through diffusion and/or evaporation processes. It is an essential concept when considering skin dryness and it is an index that is used to evaluate the repairing capacity of cosmetic products in the skin.

One objective of the present invention is a concentrate suitable for use as an active ingredient of a topically applied preparation for skin care and repair, especially with strong antioxidant action on the skin, at least enough to be used for repairing aggressions, for improving the hydrolipid layer or for the immediate and long-term hydration of the skin.

On the other hand, this strong antioxidant action is also of interest for preserving the properties of the preparation; this is to prevent the deterioration thereof by oxidation of unsaturated fatty acids, for example by excessive contact of the preparation with the air (especially when the bottles are not closed after use) or by subjecting the preparation to heat or light (which is conventionally foreseen by using coloured or opaque bottles).

Achieving good preservation without using preservatives in the form of synthetic elements or allergens in a topical preparation is of great interest in the case of applications on skin damaged by radiation therapy or by aggressive medical treatments (chemotherapy) that weaken and inflame the skin.

It is therefore also an objective of the invention a concentrate for the care and repair of the skin that does not incorporate synthetic elements or allergens for its conservation.

Description of the invention

The inventors of the present invention have disclosed that, surprisingly, the antioxidant effect especially attributed to Vitamin E in olive oil is enhanced in a way that is not caused by Hydroxytyrosol when it is added to olive oil in amounts that are not naturally present in the olive oil itself. This effect is not only adversely affected by the presence of maslinic acid in an unnatural proportion in olive oil, but it also fails to prevent said maslinic acid from showing its properties, for example, anti-inflammatory properties.

Further, the inventors have been studying different molecules from the olive tree for years and their applications in skin repair via different formulations and encapsulations to improve the effectiveness of these molecules. The inventors have revealed that the formulation with ingredients from the same origin, in this case the olive tree, show a special synergy. Thus, its repair efficiency is surprisingly accentuated compared to other mixtures / formulations with components of different origin.

That is why, according to claim 1, the invention relates to an olive oil-based concentrate to be used as an active ingredient of a topically applied preparation for skin care which comprises olive oil with an ORAC factor of at least 8 ($\mu$mol Trolox equivalents/g) which is enriched with at least one extract from the olive fruit rich in maslinic acid; and with at least one extract also from the olive fruit or leaf rich in Hydroxytyrosol.

ORAC is a scale of values in which the degree of antioxidant effect of a sample is numerically recorded by means of a value measured in micromoles of Trolox equivalents (a measurement standard) per unit weight of the sample. The higher the value assigned to a sample in this index, the greater the antioxidant power thereof.

This value system, which means Oxygen Radical Absorption Capacity, was developed by Tufts University, USA, to provide fast and reliable means to determine to what extent a product can be beneficial when facing the problem of oxidative stress or oxidation process, having a margin of error of +/- 5%.

In this case, the ORAC values of the assays referred to below were obtained by spectrofluorescence and the fluorescence readings were performed in a Synergy ™ H1M model spectrofluorometer, Hybrid Multi-Mode Microplate Reader (BioTek Instruments, Inc., Winooski, VT, USA).

This olive oil-based concentrate enriched with Hydroxytyrosol and maslinic acid has an effect on skin care, especially with strong antioxidant action on the skin, of high interest for repairing aggressions, for improving the hydrolipid layer or for the immediate and long-term hydration of the skin. The concentrate of the invention can thus be used, among other purposes, for strengthening the immune system of the skin; for reducing wrinkles; for providing more luminosity and a more homogeneous tone to the skin; for accelerating healing processes and/or for improving post-scarring marks.

Advantageously, the aforementioned moisturising properties have also been shown to be especially effective for sensitive skin (dry, reactive, sensitised or tight skin), as shown below.

The aforementioned effects are also noticeable at small doses. Thus, in a variant of the invention, the amount of maslinic acid present in the concentrate is at least 0.040% by weight of the concentrate; and the amount of Hydroxytyrosol in the concentrate is at least 0.010% by weight of the concentrate. Preferably, the amount of maslinic acid present in the concentrate is from 0.045% to 1% by weight of the concentrate; and the amount of Hydroxytyrosol in the concentrate is from 0.010% to 2% by weight of the concentrate and more preferably between 0.020% and 1% by weight of the concentrate.

According to a variant of the invention, the extract rich in Hydroxytyrosol has a richness of at least 20% in Hydroxytyrosol.

According to a variant of the invention, the extract rich in maslinic acid has a richness of at least 60% in maslinic acid, preferably between 60% to 85%.

It is also the case that the notable multiplying effect of the antioxidant properties of olive oil is such that the invention allows for the formulation of dermocosmetic and/or dermo repair preparations, for example, in the form of creams, wherein the concentrate of the invention is the active ingredient with a presence of only between 1% and 4%, and preferably between 1% and 3% by weight of the preparation.

The present invention thus also relates to a dermocosmetic and dermo repair preparation with these features, comprising between 1% and 3% by weight of the olive oil-based concentrate enriched with Hydroxytyrosol and maslinic acid.

Advantageously, the effectiveness of the concentrate discloses an alternative for topically applied formulations and preparations that use synthetic antioxidants such as butylated hydroxytoluene (BHT). The concentrate of the invention thus contributes to the production of more natural preparations, more compatible for use on human skin and again especially for sensitive skin.

Lastly, and no less interesting, the concentrate being a product obtained from the olive, the present invention offers a new incentive for this sector to continue to be one of the most dynamic sectors of the Spanish agri-food system that, in fact, acts as one of the fundamental driving forces of the Spanish economy.

Brief description of the drawings

Fig. 1 shows the antioxidant capabilities of a concentrate according to the invention;

Fig. 2 shows from what order the enhancing effect of the antioxidant properties of olive oil becomes evident in a concentrate according to the invention;

Figs. 3 and 4 illustrate the effectiveness of a concentrate according to the invention, as well as a preparation comprising only 2% by weight of the concentrate as an active ingredient, in repairing the skin that is reflected in the improvement of the Erythema rate thereof and the improvement of the TEWL index thereof, respectively;

Figs. 5 and 6 illustrate the hydration kinetics of normal skin and sensitive skin, respectively, of two dermocosmetic and repair preparations comprising 1% and 2% by weight of an olive oil-based concentrate according to the invention.

Detailed description of the invention

The surprising enhancing effect of the antioxidant properties of an additivated olive oil according to the present invention becomes plausible in the following concentrate, which exemplifies the invention.

Table 1: Formulation of a concentrate according to a variant of the invention.

| Raw material | Ingredients of the concentrate (INCI / CTFA) | % in Raw Material | % Ing. |
|---|---|---|---|
| OA OMEGA 9 [(1)] | OLEA EUROPAEA (OLIVE) FRUIT OIL | 100.00% | 99.790% |
| OA MA 75 [(2)] | OLEA EUROPAEA (OLIVE) FRUIT EXTRACT | 100.00% | 0.085% |
| OA HT ECO 20% [(3)] | OLEA EUROPAEA (OLIVE) LEAF EXTRACT | 100.00% | 0.125% |
| (1) Extra virgin olive oil (Empeltre variety) (2) Extract from the olive fruit with a purity of 60% in Maslinic acid. (3) Extract from the olive leaf with a purity of 20% in Hydroxytyrosol. | | | |

The concentrate is obtained by adding a starting oil rich in Vitamin E and with high antioxidant power with 850 ppm of OA MA 75 and 1,250 ppm of OA HT ECO 20%, of which the total concentration in the concentrate obtained is therefore:

- 510 ppm maslinic acid
- 250 ppm Hydroxytyrosol

The following Table 2 compares the antioxidant capacity on the ORAC scale of several olive oils, including the olive oil selected to obtain the concentrate. Note that the ORAC value of the concentrate is 10 times higher than that of the aforementioned starting oil, which reveals an unexpected increase in antioxidant capacity, taking into account that the presence of Hydroxytyrosol (by way of addition) is only 250 ppm. That is, with very little addition of Hydroxytyrosol the antioxidant capacity of the starting oil is multiplied significantly, having selected a starting oil with an ORAC value greater than 8 and in this case, specifically with an ORAC value of $9.56 \pm 0.70$.

Table 2: Significant increase in the ORAC value of olive oil rich in Vitamin E with small addition of extract rich in Hydroxytyrosol.

|  | ORAC ($\mu$mol Trolox equivalent/g) |
|---|---|
| Refined olive oil | 0.5 to 1.5 |
| Olive oil | 0.5 to 2 |
| Virgin olive oil or extra virgin olive oil | 2 to 6.5 |
| Extra quality olive oil | 9 to 10 |
| **Extra virgin olive oil (Empeltre variety)** | **$9.56 \pm 0.70$** |
| **Concentrate** | **$102.41 \pm 22.07$** |

The enhancing effect of the antioxidant properties of the starting olive oil can also be corroborated by comparing the antioxidant capacity of one same neutral medium that it incorporates in equal concentrations (100 ppm): in a first case, olive oil (which provides only the intrinsic antioxidant properties of the mentioned olive oil); in a second case, a synthetic antioxidant, specifically, butylated hydroxytoluene (BHT); and in a third case, a concentrate according to the invention (in the version of Table 1), respectively.

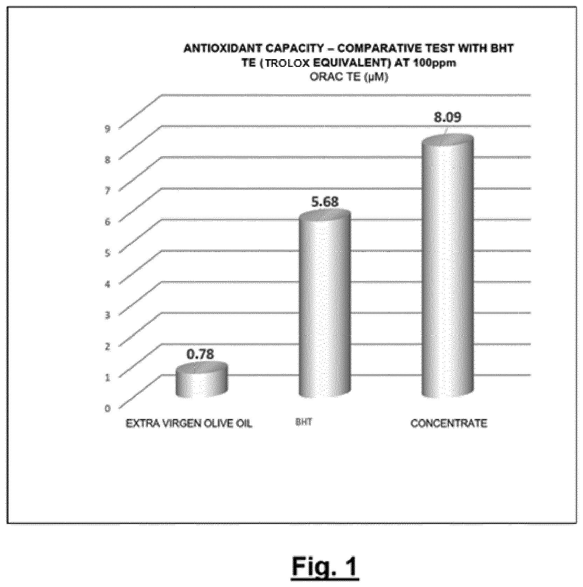

Fig. 1 shows this comparative test in which the antioxidant capacity has been measured in Trolox equivalent at the same concentrations (100 ppm).

It can be seen that the antioxidant potential is greater in the third case, that is, in the neutral medium that incorporates a concentrate according to the invention.

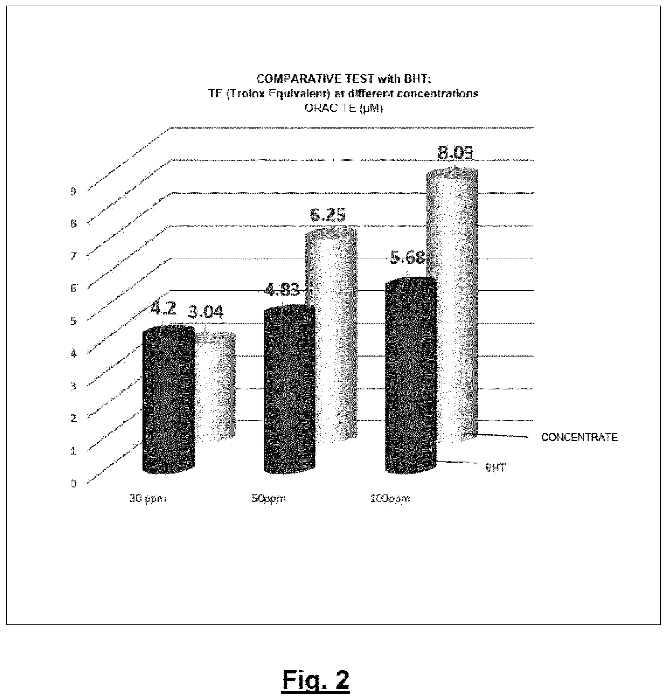

The incidence of the concentration of the ingredients that provide Hydroxytyrosol in an unnatural way to the neutral media of Fig. 1 is shown in Fig.2, having measured the antioxidant potential for different concentrations.

These tests show that the enhancing effect of the antioxidant properties of the additivated olive oil for obtaining a concentrate according to the invention are noticed for small concentrations, specifically, from concentrations of 50 ppm of the concentrate in the medium. From these concentrations the antioxidant potential of the medium that incorporates the concentrate is even greater than that resulting from directly adding the medium with butylated hydroxytoluene (BHT).

The effectiveness, with regards to skin care and repair, of this enhancing effect in a concentrate according to the invention, as well as a dermocosmetic and repair preparation comprising said concentrate, is revealed in *in vivo* assays.

The effectiveness is contrasted by testing the effect of the concentrate in a dermocosmetic and repair preparation using Erythema repair/anti-inflammatory rates and TEWL repair rates, as shown in Table 4.

Erythema (from the Greek erythros, meaning red) is redness of the skin or mucous membranes, caused by hyperemia (increased blood flow) in superficial capillaries. It occurs with any skin injury, infection, or inflammation and can be caused by infection, electrical treatment, acne medication, allergies, exercise, solar radiation (sunburn), photosensitization, cutaneous radiation syndrome, mercury toxicity, blister agents, niacin administration, or waxing and tweezing of the hairs or other actions causing the capillaries to dilate, resulting in redness. Erythema is also a common side effect of radiotherapy treatment due to patient exposure to ionizing radiation.

Turning to Table 4, the effect of the same placebo cream to which Hydroxytyrosol is incorporated in different ways is tested: in a first case alone, in a second case vehicled and encapsulated in an LD (Liposome Device, in the form of a vesicle mainly composed of phospholipids of natural origin organised in bilayers wherein the active ingredient is integrated into the core to facilitate the formulation and bioavailability of the active ingredient in the skin); and in a third case in which Hydroxytyrosol is incorporated via the concentrate, that is, as an additive in an olive oil with high ORAC index. Naturally, it is ensured that the placebo cream does not comprise ingredients that can plausibly boost or contribute to the skin's repairing effect. The formulation of the placebo cream used is the one shown in Table 3.

**Table 3:** Composition of the placebo cream used in the repair tests.

| | Placebo |
|---|---|
| Squalane: PHYTOSQUALAN® | 10.000% |
| Glvcervl stearate, PEG-100 Stearate: ARLACEL 165® | 2.500% |
| Stearvl alcohol: LANETTE 18® | 2.000% |
| DEMINERALISED WATER | 81.000% |
| Methylpropanediol, Caprylyl Glycol and Phenylpropanol: DERMOSOFT OMP® | 3.000% |
| Polvacrvlate Crosspolymer-11: Aristoflex Velvet® | 1.500% |

**Table 4:** *In vivo* assays of dermocosmetic and repair preparations additivated with Hydroxytyrosol alone or via the concentration according to the invention based on olive oil with high ORAC index.

| | % in a preparation | % HT in a preparation | ERITEMA repair rate[1] | TEWL repair rate [2] | HT content vs content in concentrate | Efficacy (rate/ HT content) | |
|---|---|---|---|---|---|---|---|
| **40% HT** (40% HT) | 0.2% | 0.08000% | 39.8% | 42.0% | 160 | 0.25 | 0.26 |
| | | | | | | | |
| **40% HT encapsulated in an LD** (0.4% HT) | 1.0% | 0.00400% | 33.1% | 39.2% | 8 | 4.1 | 4.8 |
| | | | | | | | |

(continued)

| | % in a preparation | % HT in a preparation | ERITEMA repair rate[1] | TEWL repair rate [2] | HT content vs content in concentrate | Efficacy (rate/ HT content) | |
|---|---|---|---|---|---|---|---|
| **Placebo** (0.0% HT) | - | 0.00000% | 3.4% | 4.4% | 0 | - | - |
| | | | | | | | |
| **Concentrate** | 2.0% | | 43.1% | 41.5% | | 43 | 42 |
| 0.05% ML[3]<br>0.025% HT[4]<br>99.925% Oil | | 0.00050% | | | 1 | | |
| (1) Efficacy tested at 30' of application<br>(2) Efficacy tested at 30' of application<br>(3) Via extract with a minimum Maslinic acid content of 60%.<br>(4) Via extract with a minimum Hydroxytyrosol content of 20%. | | | | | | | |

It can be seen from the results shown in Table 4 that a similar repairing effect is obtained with the dermocosmetic and repair preparation according to the invention with concentrations of Hydroxytyrosol 160 times lower than with dermocosmetic and repair preparations added alone with Hydroxytyrosol extract, meaning, it is not via the olive oil-based concentrate with high ORAC index.

The repairing effect is even greater in concentrations of Hydroxytyrosol eight times lower than with the dermocosmetic and repair preparation according to the invention compared to a dermocosmetic and repair preparation added alone with encapsulated Hydroxytyrosol or LD (which is one of the preferred forms in the industry for facilitating the formulation and the bioavailability of an active ingredient in the skin).

From the point of view of efficacy, Table 4 (see Efficacy column) reveals how the Erythema and TEWL repair rates per HT (Hydroxytyrosol) molecule are both of the order of ten times higher in the case of the preparation that incorporates the concentrate of the invention with respect to the preparation additivated with 40% HT encapsulated in an LD, meaning, as it has been pointed out before, a commonly preferred route for prolonging the bioavailability of the active ingredient, that is, Hydroxytyrosol, thus improving the absorption, penetration and diffusion thereof in the skin. In other words, a cream incorporating the concentrate of the invention has a proven efficacy ten times higher than this same cream additivated with equal amounts of Hydroxytyrosol, even when it is encapsulated in an LD.

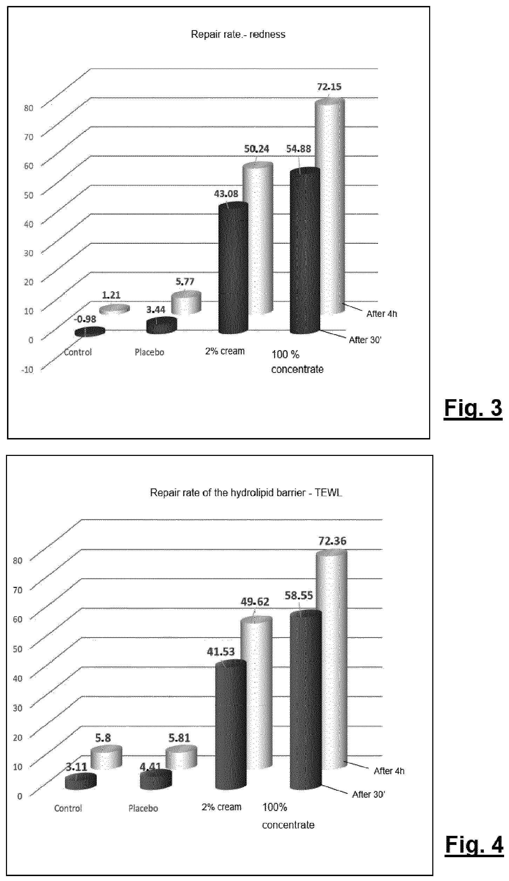

Figs. 3 and 4 graphically illustrate this efficiency when incorporating the concentrate as an active ingredient in a dermocosmetic and repair preparation according to Table 4, meaning, a 2% concentration.

Thus, Fig. 3 shows the Erythema repair rate of a dermocosmetic and repair preparation in the form of a cream with 2% of the concentrate and Fig. 4 shows the TEWL repair rate of the same dermocosmetic and repair preparation, in both cases in comparison with the placebo cream and with the 100% concentrate.

In order to evaluate the repair power of the aforementioned formulations, cutaneous stress (Erythema) has been induced by slight photoirritation (UV radiation). After 24 hours of stress induction, the different formulations were applied in the specific areas. More specifically, the skin irritation was induced with ultraviolet radiation corresponding to 1.5 times the MED (minimum erythema dose) previously calculated for each subject (a total of 20 subjects). The minimum dose that produces an evident Erythema is called "minimal erythema dose" or MED and can be evaluated by experts or instrumentally. A typical exposure time for producing Erythema in an unprotected skin is 2 minutes. Ultraviolet radiation was obtained from a 300W xenon lamp, Multiport Solar UV Simulator model 601 connected to a precision power supply, XPS400 model (Solar Light Co. Inc. Philadelphia, USA). The spectral output follows the distribution of sunlight from 290 nm to 400 nm.

The Erythema repair rate was measured with MEXAMETER®MX 18. These measurements are based on the principle of absorption. A receiver measures the light reflected by the skin so that the amount of light absorbed by the skin can be calculated.

The TEWL repair rate was measured with TEWAMETER® TM300. This measurement indicates transepidermal water loss (TEWL) and the method measures the vapour tension gradient between two electrodes located at different distances from the surface of the skin.

The readings were taken at the moments:

- [t0] (basal value): before slight light irritation (UV exposure).
- [T0']: 24 hours after stress induction (exposure to UV rays) and before the products are used.

- [T30min]: after 30 minutes of the application of the products in the specific area.
- [T4h]: after 4 hours from the application of the products in the specific area.

The repair rates are calculated using the following expressions:

$$\text{TEWL repair rate at 30' (IRt30)} = 1 - (T30'-t0) / (T0'-t0)$$

$$\text{Erythema repair rate at 30' (IRe30)} = 1 - (T30'-t0) / (T0'-t0)$$

$$\text{Average IR30} = (IRt30 + IRe30) / 2$$

$$\text{TEWL repair rate at 4h (IRe4h)} = 1 - (T4H-t0) / (T0'-t0)$$

$$\text{Erythema repair rate at 4h (IRe4h)} = 1 - (T4H-t0) / (T0'-t0)$$

$$\text{Average IR4h} = (IRt4h + IRe4h) / 2$$

From Figs. 3 and 4 not only the efficacy of the concentrate, but how it can display its practical effect in small doses is again inferred, which undoubtedly is of interest to optimise the formulations of the dermocosmetic and repair preparations that will include the concentrate (remember that a cream that incorporates only 2% by weight of the concentrate of the invention has a proven efficacy ten times higher than this same cream additivated with equal amounts of a Hydroxytyrosol extract, even when it is encapsulated in an LD - see Table 4 -).

It is possible to repair the anti-inflammatory effect of both the concentrate and the cream that incorporates the concentrate, which indicates that the properties contributed by the maslinic acid additivated in the concentrate are not adversely affected by combination with the hydroxytyrosol which is also additivated in the concentrate, or with the rest of the ingredients of the dermocosmetic and repair preparation when the concentrate is used as an active ingredient thereof.

In the case of skin damaged by radiation therapy or aggressive medical treatments (chemotherapy) that weaken and inflame the skin, the concentrated fruit of the present invention is doubly interesting. On the one hand for its anti-inflammatory and restorative effect but on the other, very important, because it does not require preservatives. For the treatment of this type of damaged skin it is essential to minimize the presence of preservatives, synthetic ingredients or allergens. The concentrate is a cosmetic in itself (as shown in Figure 3 Concentrated 100%) and also a good carrier to add other natural components. The concentrate is very rich in Omega 9, an acid present in the cell walls and therefore promotes the penetration of the actives. It is therefore the perfect solution for the development of 100% natural cosmetics, without preservatives and with high repair efficiency.

As for the hydration kinetics, several versions of the dermocosmetic and repair preparation were assayed, in the form of the placebo cream and the same placebo cream as a base and with concentrations of 1 % and 2% of the concentrate in Table 3. The formulations for the kinetic assay are shown in Table 5.

**Table 5:** Composition of the three dermocosmetic and repair preparations in the form of individual creams for the kinetic assays on skin hydration.

|  | Placebo Cream | 1% Cream | 2% Cream |
|---|---|---|---|
| Squalane: PHYTOSQUALAN® | 10.000% | 10.000% | 10.000% |
| Glyceryl stearate, PEG-100 Stearate: ARLACEL 165® | 2.500% | 2.500% | 2.500% |
| Stearvl alcohol: LANETTE 18® | 2.000% | 2.000% | 2.000% |
| DEMINERALISED WATER | 81.000% | 80.000% | 79.000% |
| Methylpropanediol, Caprylyl Glycol and Phenylpropanol: DERMOSOFT OMP ® | 3.000% | 3.000% | 3.000% |

(continued)

| | Placebo Cream | 1% Cream | 2% Cream |
|---|---|---|---|
| Polyacrylate Crosspolymer-11: Aristoflex Velvet® | 1.500% | 1.500% | 1.500% |
| Concentrate | **0.000%** | **1.000%** | **2.000%** |

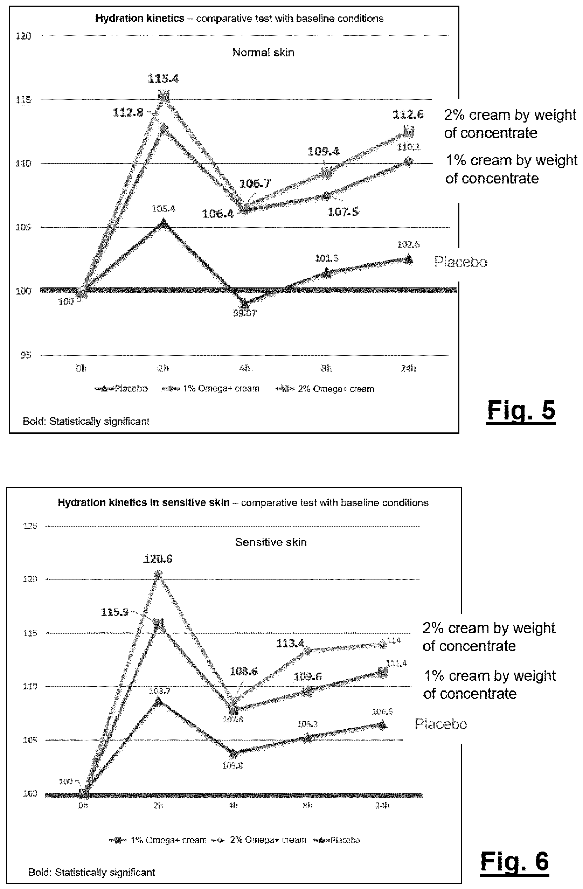

The results obtained are illustrated graphically in Figs. 5 and 6. They show a graphic representation of the hydration kinetics of the skin quantified through the corneometer for 24 hours after a single application of the placebo cream and of the creams with concentrations of 1% and 2% of the concentrate on the forearms of 20 volunteers with normal skin (Fig. 5) and on the forearms of 13 volunteers affected with sensitive skin (Fig. 6).

From this Fig. 5 a remarkable increase in the hydration of the skin is inferred after a single application of the creams comprising a concentrate according to the invention with respect to the placebo cream. Likewise, a bimodal dynamic of interest is observed in which an immediate increase in hydration two hours after application and a progressive long-term increase (8h to 24h) are noticed.

Fig. 6 reveals an unexpected improved effect for users affected with sensitive skin. Indeed, it is important to note that although the effect of the cream with concentration at 2% of the concentrate shows a value 15 points above the reference value in the hydration peak, this value is 20 points above the reference in the case of sensitive skin, which means approximately 5 points of improvement in the reparative response of the cream in sensitive skins.

The direct topical application of an olive-oil based concentrate according to the invention is also envisaged. For example, it is an embodiment of the present invention an olive-oil based concentrate for skin care and repair consisting essentially of olive oil with an ORAC factor of at least 8 ($\mu$mol Trolox equivalents/g) which is enriched with at least one extract from the olive fruit rich in maslinic acid; and with at least one extract also from the olive fruit or leaf rich in Hydroxytyrosol, wherein the amount of maslinic acid present in the concentrate is at least 0.040% by weight of the concentrate; and the amount of Hydroxytyrosol in the concentrate is at least 0.010% by weight of the concentrate.

The expression "consisting essentially of" is to be interpreted as allowing the presence of amounts of other components in addition to the mandatory components, provided that the essential characteristics of the claimed composition are not materially affected by their presence. These other components, apart from possible impurities, are in the following example a maslinic acid powder supplement (such as 85% pure) and perfume, in a total amount of less than 2% in weigh.

**Table 6:** Composition of a topical olive-oil based concentrate for skin care and repair.

| Ingredients | % Ing. |
|---|---|
| Concentrate (as exemplified in Table 4: 99.925% Oil; 0.05% ML; 0.025% HT) | 98,80% |
| MASLINIC POWDER (Olea Europeae - olive - fruit extract) | 0,20% |
| Perfume (fragrance) | 1% |

**Claims**

1. An olive-oil based concentrate to be used as an active ingredient of a topically applied preparation for skin care and repair **characterised in that** it comprises olive oil with an ORAC factor of at least 8 ($\mu$mol Trolox equivalents/g) which is enriched with at least one extract from the olive fruit rich in maslinic acid; and with at least one extract also from the olive fruit or leaf rich in Hydroxytyrosol.

2. The olive oil-based concentrate according to claim 1, **characterised in that** the extract rich in Hydroxytyrosol has a richness of at least 20% in Hydroxytyrosol.

3. The olive oil-based concentrate according to claims 1 or 2, **characterised in that** the extract rich in maslinic acid has a richness of at least 60% in maslinic acid.

4. The olive oil-based concentrate according to any one of the preceding claims, **characterised in that**

    - the amount of maslinic acid present in the concentrate is at least 0.040% by weight of the concentrate; and **in that**
    - the amount of Hydroxytyrosol in the concentrate is at least 0.010% by weight of the concentrate.

5. The olive oil-based concentrate according to the preceding claim, **characterised in that**

   - the amount of maslinic acid present in the concentrate is between 0.045% and 1% by weight of the concentrate; and **in that**
   - the amount of Hydroxytyrosol in the concentrate is between 0.020% and 1% by weight of the concentrate.

6. The olive oil-based concentrate according to any of the preceding claims for skin care and repair, consisting essentially of olive oil with an ORAC factor of at least 8 ($\mu$mol Trolox equivalents/g) which is enriched with at least one extract from the olive fruit rich in maslinic acid; and with at least one extract also from the olive fruit or leaf rich in Hydroxytyrosol.

7. A topical preparation for skin care and repair comprising an olive oil-based concentrate according to any one of the preceding claims.

8. A topical preparation for skin care and repair comprising between 1% and 3% by weight of an olive oil-based concentrate according to any one of the claims 1 to 6.

9. The topical preparation for skin care and repair according to the preceding claim in the form of a cream or a gel.

10. The use of the oil-based concentrate according to any one of claims 1 to 6 for the preparation of a dermocosmetic and repair product.

11. The oil-based concentrate according to any of the claims 1 to 6 or the topical preparation according to any of the claims 7 to 9 for Erythema repair.

**ANTIOXIDANT CAPACITY – COMPARATIVE TEST WITH BHT
TE (TROLOX EQUIVALENT) AT 100ppm**
ORAC TE (µM)

## Fig. 1

**COMPARATIVE TEST with BHT:**
TE (Trolox Equivalent) at different concentrations
ORAC TE (µM)

**Fig. 2**

**Fig. 3**

**Fig. 4**

**Fig. 5**

**Fig. 6**

Europäisches
Patentamt

European
Patent Office

Office européen
des brevets

# EUROPEAN SEARCH REPORT

Application Number

EP 19 38 2848

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 2 722 050 A2 (AGENCIA PUBLICA EMPRESARIAL SANITARIA HOSPITAL ALTO GUADALQUIVIR [ES]) 23 April 2014 (2014-04-23) | 1,6-11 | INV. A61K8/34 A61K8/63 A61K8/97 |
| Y | * paragraphs [0001], [0015] - [0020]; claims * | 1-11 | A61K36/36 A61P17/00 A61Q19/00 |
| Y | RO 128 713 A2 (ZBUCHEA ANDREI-GHEORGHE [RO]) 30 August 2013 (2013-08-30) * paragraphs [0001], [0002], [0004]; claims * | 1-11 | A61K8/92 A61K8/9789 |
| Y | ES 2 389 816 A1 (OLMO PEINADO JOSE MARIA [ES]; RUIZ RUEDA JESUS TOMAS [ES]) 2 November 2012 (2012-11-02) * paragraphs [0003], [0004], [0012] - [0015] * | 1-11 | |
| Y | V. SÁNCHEZ DE MEDINA ET AL: "Characterization of Refined Edible Oils Enriched with Phenolic Extracts from Olive Leaves and Pomace", JOURNAL OF AGRICULTURAL AND FOOD CHEMISTRY, vol. 60, no. 23, 1 June 2012 (2012-06-01), pages 5866-5873, XP055645295, US ISSN: 0021-8561, DOI: 10.1021/jf301161v * the whole document * | 1-11 | TECHNICAL FIELDS SEARCHED (IPC) A61K A61P A61Q |

-/--

The present search report has been drawn up for all claims

| Place of search | Date of completion of the search | Examiner |
|---|---|---|
| Munich | 28 November 2019 | Heller, Dorothée |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 19 38 2848

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | SALTA F N ET AL: "Oxidative stability of edible vegetable oils enriched in polyphenols with olive leaf extract", FOOD SCIENCE AND TECHNOLOGY INTERNATIONAL, SAGE PUBLICATIONS, NEW YORK, NY, US, vol. 13, no. 6, 1 January 2007 (2007-01-01), pages 413-421, XP002541264, ISSN: 1082-0132, DOI: 10.1177/1082013208089563 * abstract * ----- | 1-11 | |

TECHNICAL FIELDS
SEARCHED       (IPC)

The present search report has been drawn up for all claims

| Place of search | Date of completion of the search | Examiner |
|---|---|---|
| Munich | 28 November 2019 | Heller, Dorothée |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 19 38 2848

This annex lists the patent family members relating to the patent documents cited in the above-mentioned European search report.
The members are as contained in the European Patent Office EDP file on
The European Patent Office is in no way liable for these particulars which are merely given for the purpose of information.

28-11-2019

| Patent document cited in search report | | Publication date | Patent family member(s) | Publication date |
|---|---|---|---|---|
| EP 2722050 | A2 | 23-04-2014 | EP 2722050 A2<br>ES 2395539 A1<br>WO 2012172150 A2 | 23-04-2014<br>13-02-2013<br>20-12-2012 |
| RO 128713 | A2 | 30-08-2013 | NONE | |
| ES 2389816 | A1 | 02-11-2012 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1295587 A
- ES 2462565